(19) Europäisches Patentamt

European Patent Office

Office européen des brevets

(11) Publication number: **0 321 180 B1**

(12) **EUROPEAN PATENT SPECIFICATION**

(45) Date of publication of patent specification: **29.07.92** (51) Int. Cl.⁵: **A61K 7/18**

(21) Application number: **88311769.9**

(22) Date of filing: **13.12.88**

(54) **Oral compositions.**

(30) Priority: **17.12.87 JP 319537/87**

(43) Date of publication of application:
**21.06.89 Bulletin 89/25**

(45) Publication of the grant of the patent:
**29.07.92 Bulletin 92/31**

(84) Designated Contracting States:
**AT BE CH DE ES FR GB IT LI NL SE**

(56) References cited:
**FR-A- 2 406 437**

(73) Proprietor: **LION CORPORATION**
**3-7, Honjo 1-chome**
**Sumida-ku Tokyo(JP)**

(72) Inventor: **Ishikawa, Masao**
**No. 15-3-705, Wada 1-chome Hodogaya-ku**
**Yokohama-shi Kanagawa-ken(JP)**
Inventor: **Shibuya, Koji**
**No. 3707, Higashikoiso Oiso-machi**
**Naka-gun Kanagawa-ken(JP)**

(74) Representative: **Stuart, Ian Alexander et al**
**MEWBURN ELLIS & CO. 2/3 Cursitor Street**
**London EC4A 1BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

This invention relates to oral cavity compositions having improved mouth odor suppression. The term "oral cavity composition" is hereinbelow abbreviated to "oral composition ".

It is known in the art to blend such agents for suppressing mouth odors as chlorhexidine and sodium copper chlorophyllin in oral compositions such as dentifrices. However, chlorhexidine cannot fully exert its mouth odor suppressing effect in dentifrices or other oral compositions containing an anionic or nonionic surface active agent. Because of its own color, sodium copper chlorophyllin sometimes causes the oral compositions to be colored and is thus undesirable to blend in white toothpaste.

It is also known that copper compounds including copper gluconate, copper citrate and copper long chain alkyloyl sarcosines are effective in reducing mouth odors. A number of oral compositions containing such copper compounds are known from U.S Patent Nos. 3,044,939, 3,565,933, 3,655,868, and 4,112,066 and Japanese Patent Application Kokai Nos. 56-169619, 56-169620, 61-37720, and 61-143317.

These copper compounds, however, do not fully exert their mouth odor reducing effect when blended in relatively small amounts. The copper compounds must be blended in relatively large amounts to assure their effect, but at the sacrifice of a feel to the mouth because copper is astringent.

It is also known from Japanese Patent Application Kokai No. 62-142109 to use copper gluconate in combination with sodium monofluorophosphate. We have found that the combined use of copper gluconate with sodium monofluorophosphate gives relatively higher mouth odor suppression than the use of copper gluconate alone, but is not fully effective in relatively small amounts.

Therefore, an object of the present invention is to provide a novel and improved oral composition exhibiting increased mouth odor suppression.

We have investigated an oral composition which exhibits improved mouth odor suppression even when a deodorizing agent is used in a small amount sufficient to eliminate its influence on the flavor or preparation of the composition. We have found that when copper gluconate, a fluorine compound, and an alkali metal salt of an alkyl sulfate having 8 to 18 carbon atoms are used in admixture, these compounds act in a synergistic manner so that increased mouth odor suppression is accomplished even when each ingredient is contained in a low concentration.

The oral composition of the present invention provides increased mouth odor suppression due to the combined use of copper gluconate, a fluorine compound, and an alkali metal salt of an alkyl sulfate having 8 to 18 carbon atoms. Since the required effect is achieved with a low concentration of each ingredient, the amount of each ingredient can be reduced to such a level as to give no influence on the flavor or preparation of the composition. The use of a mixture of these three ingredients avoids a reduction of feel to the mouth due to astringency, unpleasant taste and juice effect resulting from the presence of large amounts of these ingredients and leaves a greater degree of freedom in flavoring and preparation of the composition.

As described above, the present invention provides an oral cavity composition comprising in admixture, copper gluconate, a soluble fluorine compound, and an alkali metal salt of an alkyl sulfate having 8 to 18 carbon atoms; wherein said copper gluconate, fluorine compound and alkali metal salt constitute a synergistic mixture for mouth odor suppression.

In the practice of the present invention, copper gluconate is preferably blended in an amount of 0.00001 to 2% by weight, more preferably 0.001 to 0.5% by weight based on the total weight of the composition.

The fluorine compounds used herein include sodium fluoride, sodium monofluorophosphate, stannous fluoride, and stannic fluoride. They may be used alone or in admixture of two or more. They are preferably blended in an amount of 0.001 to about 1% by weight, more preferably 0.01 to 0.5% by weight based on the total weight of the composition.

The alkali metal salts of alkyl sulfates having 8 to 18 carbon atoms used herein are alkali metal salts of ($C_8$-$C_{18}$) alkyl sulfates including sodium lauryl sulfate and sodium myristyl sulfate. They may be used alone or in admixture of two or more. They are preferably blended in an amount of 0.0001 to 3% by weight, more preferably 0.001 to 2% by weight based on the total weight of the composition.

The composition of the present invention may contain in addition to the copper gluconate, copper citrate or an alkali metal salt of copper citrate such as disodium copper (II) citrate ($Na_2CuC_6H_4O_7.xH_2O$), dilithium copper (II) citrate ($Li_2CuC_6H_4O_7.xH_2O$), and dipotassium copper (II) citrate ($K_2CuC_6H_4O_7.xH_2O$), or an inorganic copper compound such as copper sulfate ($CuSO_4.xH_2O$), copper hydroxide ($Cu(OH)_2.xH_2O$), and copper chloride ($CuCl_2.xH_2O$) in an amount of up to about 2% by weight, more preferably about 0.00001 to about 2% by weight, especially up to about 0.5% by weight, most preferably about 0.0005 to about 0.5% by weight based on the total weight of the composition.

The oral composition of the present invention may be prepared in any desired forms including paste,

2

liquid, powder and solid depending on the intended application as dentifrices such as toothpaste, toothpowder, and liquid dentifrice, mouthwashes, troches, gargle tablets, and chewing gums. The oral composition may contain other ingredients which may be suitably chosen from commonly used ones depending on the end application. For example, in the case of dentifrices, there may be blended abrasives, binders, humectants, surface-active agents, sweeteners, flavors, preservatives, and other active agents. Although the alkali metal alkyl sulfate having 8 to 18 carbon atoms acts herein as a surface-active agent, any other surface-active agent may be additionally blended.

More specifically, the oral composition may contain an abrasive such as calcium secondary phosphate dihydrate, calcium secondary phosphate anhydride, calcium primary phosphate, calcium tertiary phosphate, calcium carbonate, calcium pyrophosphate, insoluble sodium metaphosphate, amorphous silica, crystalline silica, aluminosilicate, aluminum silicate, aluminum oxide, aluminum hydroxide, magnesium tertiary phosphate, magnesium carbonate, magnesium sulfate, titanium oxide, and resin. In preparing a dentifrice, the abrasive is preferably blended in an amount of about 1 to about 99%, especially about 10 to about 70% by weight of the composition.

Preferred among these abrasives are precipitated amorphous silicas and silicates. When an abrasive in the form of precipitated amorphous silica or silicate is combined with copper gluconate and an optional copper compound, improved mouth odor suppression is accomplished even with a substantially small amount of copper compound.

The precipitated amorphous silicas and silicates used herein may be those obtained by conventional methods, but preferably have a specific surface area of up to 120 $m^2/g$, more preferably up to 80 $m^2/g$ as measured by the BET method using nitrogen gas. A typical precipitated amorphous silica is SIDENT® 12 (manufactured and sold by Degussa Co.). Examples of the precipitated amorphous silicates used herein include precipitated aluminosilicate, zirconosilicate, borosilicate, calcium silicate, and magnesium silicate. These silicates preferably have a polyvalent metal content of up to about 20%, especially up to 10% by weight. The amount of the precipitated amorphous silica or silicate blended herein is not particularly limited although it is preferably used in an amount of about 5 to 50%, more preferably about 8 to about 35% by weight of the total composition.

The oral composition of the present invention may also contain a binder such as sodium carboxymethyl cellulose, methyl cellulose, sodium carboxymethyl hydroxyethyl cellulose, hydroxyethyl cellulose, sodium alginate, carrageenan, gum arabic, xanthane gum, tragacanth gum, karaya gum, polyvinyl alcohol, sodium polyacrylate, carboxyvinyl polymer, and polyvinyl pyrrolidone; and a humectant such as polyethylene glycol, ethylene glycol, sorbitol, glycerin, propylene glycol, 1,3-butylene glycol, xylitol, maltitol, and lactitol. The amount of the binder blended preferably ranges from 0 to about 5%, more preferably from about 0.1 to about 5% by weight of the total composition. The amount of the humectant blended preferably ranges from about 10 to about 80%, more preferably from about 30 to about 60% by weight of the total composition.

The oral composition of the present invention may further contain one or more members of anionic, nonionic, cationic, and amphoteric surface-active agents in an amount of 0 to about 5%, more preferably 0 to about 2% by weight of the composition in addition to the water-soluble alkali metal salts of alkyl sulfates. Examples of the anionic surface-active agents include sodium coconutmonoglyceride sulfonate and other water-soluble higher acid monoglyceride sulfonates derived from higher fatty acids having 10 to 18 carbon atoms; sodium salts of $\alpha$-olefin sulfonates, paraffin sulfonate, and N-methyl-N-palmitoyltauride; sodium N-lauroyl sarcosine; and sodium N-lauroyl-$\beta$-alanine. Examples of the nonionic surface-active agents include lauroyl diethanolamide and other fatty acid alkanolamides; sucrose monolaurate, sucrose dilaurate, and other sucrose fatty acid esters; polyoxyethylene sorbitan fatty acid esters, polyoxyethylene fatty acid esters, polyoxyethylene hardened castor oil derivatives, lactose fatty acid esters, lactitol fatty acid esters, maltitol fatty acid esters, and polyoxyethylene-polyoxypropylene block copolymers.

The oral composition of the present invention may further contain a sweetener such as saccharin sodium, stevioside, neohesperidyldihydrochalcone, glycyrrhizin, perillartine, and p-methoxycinnamic aldehyde; a flavor; a preservative; and an active ingredient such as dextranase, protease, lysozyme, lytic enzyme, mutanase, sorbic acid, $\beta$-glycyrrhetinic acid, hinokitiol, chlorhexidine, alkylglycine, alkyldiaminoethylglycine hydrochloride, allantoin, epsilonaminocaproic acid, tranexamic acid, zinc chloride, zinc gluconate, zinc hydroxide, triclosan, azuren, vitamin E, water-soluble primary and secondary phosphates, cetylpyridinium chloride and other quaternary ammonium compounds, sodium chloride, crude drug extracts.

Among these additives, it is particularly preferred to add at least one flavor selected from the group consisting of cardamom oleo-resin, laurel oleo-resin, nutmeg oleo-resin, pimento oleo-resin, fennel oleo-resin, anise oleo-resin, bergamot oil, anethole, orange oil, lemon oil, and lime oil. These flavors are effective to control the astringency of copper gluconate, copper citrate and alkali metal salts thereof. The flavor may preferably be added in an amount of about $10^{-5}$ to about 1%, more preferably about 0.0005 to about 0.2% by weight of the total composition.

It is also recommended to use in addition to the above-mentioned copper compound at least one bactericide selected from the group consisting of chlorhexidine gluconate, chlorhexidine hydrochloride, cetyl pyridinium chloride, benzethonium chloride, benzalkonium chloride, decalinium chloride, alkyldiaminoethylglycine hydrochloride, and isopropylmethylphenol. The copper compound synergistically assists the bactericide in exerting its bactericidal activity while increasing the retention of mouth odor suppression effect, assuring that a very small amount of the bactericide used effectively controls mouth odors. The bactericide may preferably be added in an amount of about $10^{-5}$ to about 1%, more preferably about $10^{-4}$ to about 0.3% by weight of the total composition. The amount of the bactericide blended may be substantially reduced because the copresence of the bactericide and the copper compound enhances bactericidal activity and continuing mouth odor suppression in a synergistic manner.

It is also recommended to use in addition to the above-mentioned copper compound a solvent extract from a labiatae or myrtaceae family plant. These two ingredients synergistically act to substantially deodorize volatile sulfur compounds such as methyl mercaptan. The deodorizing effect quickly appears particularly when the two ingredients are prepared into aqueous solution. A serendipitious advantage from the combined use is increased retentivity of deodoring effect. Examples of the labiatae and myrtaceae family plants from which a solvent extract is obtained are given below.

I. Laviatae family plants

Firmiana platanifolia Schott et Endl.
Isodon longitubus Kudo
Salvia japonica Thumb.
Mosia punctulata Nakai
Stachys Riederi Chamisso
Isodon umbrosus Hara
Thymus quinquecostatus Celak
Prunella vulgaris L.
Perilla frutescens Britton var. japonica Hara
Lamium album L. var. barbatum Frachet et S.
Mentha spicata L. var. crispa Benth.
Origanum vulgare (oregano)
Ajuga ciliata Bunge
Glechlma hederacea L.
Isodon Kameba Okuyama
Agastache rugosa O. Kuntze
Salvia nipponica Miq.
Ajuga decumbens Thunb.
Clinopodium chinense O. Kuntze var. parviflorum Hara
Schizonepeta tenuifolia Briquet var. japonica Kigawa
Salvia officianalis L.
Perilla frutescens Britton var. acuta Kudo (perilla)
Keiskea japonica Miq.
Lycopus lucidus Turcz.
Chelonopsis moschata Miq.
Ajuga nipponensis Makino
Satureia spp.
Thymus vulgaris
Scutellaria indica L.
Salvia miltiorrhiza Bunge
Nepeta Cataria L.
Stachys Sieboldi Mig.
Perilla frutescens viridi-crispa Makino
Comanthosphace japonica S. Moore
Clinopodium gracile O. Kuntze
Thymus vulgaris (thyme)
Scutellaria baicalensis Georgi
Elshotizia ciliata Hylander

4

Teucrium japonicum Houtt
Marrubium vulgare L.
Mentha arvensis L. var. piperascens Malinv. (mentha)
Ocymum basilicum L. (basil)
Pogostemon cablin Hara
Isodon japonicus Hara
Salvia splendents Ker
Hyssopus officianalis L.
Lamium purpureum L.
Mosla dianthre Maxim
Mentha piperita
Lamium amplexicaule L.
Origanum spp. (marjoram)
Rosmarinus officinalis L.
Dysophylla verticillata Benth.
Nepeta subscenssili Maxim.
Salvia plebeia R. Brown
Leonurus sibiricus L.
Melissa officinalts L.
Monarda fistulosa
Lamium humile Maxim.
Mosla japonica Maxim.
Isodon inflexus Kudo
Meehania urthicifolia Makino
Lavandula spp. (lavender)
Amethyste caerulea L.
Rosmarinus officinalis L. (rosemary)
Salvia Officinalis L. (sage)

II. Myrtaceae family plants

Syzygium aromaticum (clove)
Eucalyptus globulus L. (eucalyspus)
Myrtaceae Pimenta officinalis (allspice)
Myrtaceae psidium gua
Rhodomyrtus tomentosa Hassk

In obtaining a solvent extract from these plants, either raw or dry plants may be used. For ease of operation, dry finely crashed plant is used for Solvent extraction. Extraction may be carried out by a conventional method as by immersing the plant in a heated solvent or by using a Soxhlet's extractor. If desired, hydrolysis may be carried out. The solvents used herein include one or more polar solvents such as water, aqueous acid solutions, aqueous inorganic salt solutions, aqueous alkali solutions, ethyl ether, ethylene chloride, dioxane, acetone, methanol, ethanol, n-butanol, ethyl acetate, and propylene glycol; and one or mor non-polar solvents such as n-hexane, petroleum ether, ligroin, cyclohexane, carbon tetrachloride, chloroform, dichloromethane, 1,2-dichloroethane, toluene, and bezene. A mixture of polar and non-polar solvents is also useful.

The extract resulting from the above-mentioned procedure is generally blended with the remaining ingredients of the composition after the solvent has been distilled off. When the solvent is water, ethanol or another safe one, the extract may be added to the remaining ingredients of the composition without removal of the solvent. The solvent extract from a plant may be blended in any desired amount, preferably in an amount of about 0.001 to about 2%, more preferably about 0.005 to about 1% by weight of the total composition.

The oral composition preferably has a pH in the range of 5 to 10 when it is of liquid or paste type.

Experiments were carried out to demonstrate the effectiveness of the oral composition of the present invention.

Experiment 1

A sterilized culture medium was prepared by adding 0.5 ml of a sample solution containing copper gluconate, sodium lauryl sulfate, and sodium monofluorophosphate in the concentration (% by weight) reported in Table 1 to 4.5 ml of a Todd Hewitt broth culture medium containing 0.05% by weight cysteine (manufactured by DIFCO Co.).

To 4.5 ml of the sterilized culture medium was added 0.1 ml of pre-cultured Fusobacterium nucleatum 1436 having an optical density (O.D.) of 1.0. The medium was cultured for 2 days at 37°C under anaerobic conditions (80% $N_2$, 10% $H_2$, and 10% $CO_2$).

At the end of cultivation, the extent of growth of the bacterium in each medium was determined by measuring the absorbance at a wavelength of 550 nm. The results are also shown in Table 1.

As a control, germ-free water was added as a sample solution to a sterilized culture medium to which 0.1 ml of pre-cultured Fusobacterium nucleatum 1436 (O.D. = 1.0) was added for anaerobic cultivation.

Table 1

| Sample No. | Copper gluconate (%) | Sodium lauryl sulfate (%) | Sodium monofluorophosphate (%) | Absorbance (%) |
|---|---|---|---|---|
| 1 | 0.005 | - | - | 0.75 |
| 2 | 0.005 | 0.001 | - | 0.60 |
| 3 | 0.005 | - | 0.01 | 0.51 |
| 4 | 0.005 | 0.001 | 0.01 | 0.12 |
| 5 | 0.005 | 0.001 | 0.05 | 0 |

As seen from Table 1, the combined use of copper gluconate, sodium monofluorophosphate and sodium lauryl sulfate (sample Nos. 4 and 5) provides satisfactory mouth odor suppression even though each ingredient is present in a low concentration.

Similar results are obtained when sodium monofluorophosphate was replaced by sodium fluoride, stannous fluoride, and stannic fluoride in the above experiment.

Experiment 2

Toothpastes having the formulation shown in Table 2 were prepared and observed for appearance and deodorizing power. The results are shown in Table 2. The measurement of deodorizing power is described below.

Measurement of deodorizing power

A test tube having an internal volume of 15 ml was charged with 1 gram of the toothpaste, and 0.1M phosphate buffer was added to pH 7.5. Immediately after 0.5 ml of a 10 vol% ethanol aqueous solution containing 1 µg/ml of methyl mercaptan was added, the tube was closed with a silicone rubber plug and vigorously shaken for one minute. The tube was allowed to stand for 6 minutes at 37°C before a gas chromatographic syringe was punctured into the plug to introduce 5 ml of air into the tube. The tube was again vigorously shaken for 30 seconds. Then 5 ml of gas was collected from the head space through the syringe and immediately introduced into a gas chromatograph, Model 163 (manufactured by Hitachi Ltd., Japan). The integrated count of methyl mercaptan was measured and percent deodorization was calculated therefrom according to the following formula:

percent deodorization = (C - S)/C x 100%

wherein C is an integrated count of Control (water) and S is an integrated count of a sample.

Table 2

| Sample No. | 6 | 7 | 8 | 9 | 10 |
|---|---|---|---|---|---|
| Composition (% by weight) | | | | | |
| Copper gluconate | 0.005 | 0.0025 | 0.005 | 0.005 | 0.005 |
| Copper citrate | - | 0.0025 | - | - | - |
| Sodium monofluorophosphate | 0.1 | 0.1 | 0.1 | - | - |
| Amorphous silica[1] | 30.0 | 30.0 | - | - | - |
| Zirconosilicate[2] | - | - | 30.0 | - | - |
| Silica gel[3] | - | - | - | - | 30.0 |
| Calcium hydrogen phosphate dihydrate | - | - | - | 45.0 | - |
| Propylene glycol | 2.0 | 2.0 | 2.0 | 2.0 | 2.0 |
| Sorbitol solution (60%) | 35.0 | 35.0 | 35.0 | 25.0 | 35.0 |
| Sodium carboxymethyl cellulose | 1.0 | 1.0 | 1.0 | 1.0 | 1.0 |
| Sodium saccharin | 0.1 | 0.1 | 0.1 | 0.1 | 0.1 |
| Flavor | 1.0 | 1.0 | 1.0 | 1.0 | 1.0 |
| Sodium lauryl sulfate | 1.5 | 1.5 | 1.5 | 1.5 | 1.5 |
| Pure water | b | a | l a | n c | e |
| Total | 100 | 100 | 100 | 100 | 100 |
| Evaluation | | | | | |
| Appearance | white | white | white | white | white |
| Deodorization (%) | 100 | 100 | 100 | 23 | 38 |

1) precipitated amorphous silica having a specific surface area of 65 $m^2$/g as measured by the BET method

2) precipitated zirconosilicate having a specific surface area of 20 $m^2$/g as measured by the BET method

3) specific surface area 450 $m^2$/g as measured by the BET method

As seen from Table 2, noticeable deodorizing effect is obtained when precipitated amorphous silica or silicate was used in combination with copper gluconate and copper citrate (Sample Nos. 6-8).

Experiment 3

Toothpastes having the following formulation were prepared and evaluated for astringency after tooth brushing therewith using a panel of ten experts. The criterion for evaluation is given below. The results are shown in Table 3. It is to be noted that the result is represented as the number of experts who gave the rating under each heading.

7

| Toothpaste formulation | % by weight |
|---|---|
| Calcium secondary phosphate dihydrate | 50 |
| Glycerin | 20 |
| Sodium carboxymethyl cellulose | 1 |
| Sodium lauryl sulfate | 1 |
| Sodium monofluorophosphate | 0.1 |
| Sodium saccharin | 0.1 |
| 1-menthol | 0.5 |
| Carvone | 0.5 |
| Flavor of Table 3 | shown in Table 3 |
| Copper gluconate | 0.1 |
| Water | balance |
| Total | 100.0% |

| Evaluation | Rating |
|---|---|
| Very astringent | +3 |
| Astringent | +2 |
| Somewhat astringent | +1 |
| Not astringent | 0 |

Table 3

| Sample No. | Flavor | | +3 | +2 | +1 | 0 |
|---|---|---|---|---|---|---|
| 11 | - | | 6 | 4 | 0 | 0 |
| 12 | Anethole | 0.1% | 0 | 0 | 2 | 8 |
| 13 | Cardamom oleo-resin | 0.001% | 0 | 1 | 2 | 7 |
| 14 | Nutmeg oleo-resin | 0.005% | 0 | 0 | 3 | 7 |
| 15 | Bergamot oleo-resin | 0.05% | 0 | 1 | 3 | 6 |
| 16 | Orange oil | 0.05% | 0 | 0 | 4 | 6 |
| 17 | Lemon oil | 0.05% | 0 | 0 | 3 | 7 |

Experiment 4

Mouthwashes having the following formulation were prepared and evaluated for astringency after washing the mouth therewith using a panel of ten experts. The results are shown in Table 4.

8

| Mouthwash formulation | % by weight |
|---|---|
| Ethanol | 20 |
| Glycerin | 10 |
| Polyoxyethylene hardened castor oil | 1 |
| Sodium lauryl sulfate | 0.02 |
| Sodium saccharin | 0.05 |
| Stannous fluoride | 0.005 |
| Peppermint oil | 0.3 |
| Flavor of Table 4 | shown in Table 4 |
| Copper gluconate | 0.05 |
| Water | balance |
| Total | 100.0% |

Table 4

| Sample No. | Flavor | | + 3 | + 2 | + 1 | 0 |
|---|---|---|---|---|---|---|
| 18 | - | | 7 | 3 | 0 | 0 |
| 19 | Lime oil | 0.02% | 0 | 0 | 3 | 7 |
| 20 | Fennel oleo-resin | 0.005% | 0 | 0 | 4 | 6 |
| 21 | Laurel oleo-resin | 0.005% | 0 | 1 | 3 | 6 |
| 22 | Pimento oleo-resin | 0.005% | 0 | 0 | 4 | 6 |
| 23 | Anise oleo-resin | 0.01% | 0 | 0 | 2 | 8 |

Experiment 5

A sterilized culture medium was prepared by adding the copper compound and bactericide whose type and amount are reported in Table 5, 5 ppm of sodium lauryl sulfate and 5 ppm of sodium monofluorophospahte to a Todd Hewitt broth culture medium containing 0.05% by weight cysteine (manufactured by DIFCO Co.).

To 4 ml of the sterilized culture medium was added 40 $\mu$l of pre-cultured Fusobacterium nucleatum 1436 having an optical density (O.D.) of 1.0. The medium was cultured for 2 days at 37°C under anaerobic conditions (80% $N_2$, 10% $H_2$, and 10% $CO_2$).

At the end of cultivation, the extent of growth of the bacterium in each medium was determined by measuring the absorbance at a wavelength of 550 nm. The percent growth inhibition was calculated therefrom according to the following formula:

bacterium growth inhibition = (C - s)/C x 100%

wherein C is an absorbance of Control and S is an absorbance of a sample.

As a control, the copper compound, the bactericide, sodium lauryl sulfate, and sodium monofluorophosphate were omitted from a sterilized culture medium to which 0.1 ml of pre-cultured Fusobacterium nucleatum 1436 (O.D. = 1.0) was added for anaerobic cultivation.

The results are also shown in Table 5.

Table 5

| Sample No. | Bactericide | | Bacterium growth inhibition (%) | | |
|---|---|---|---|---|---|
| | Type | Amount | No copper addition | 25 ppm copper gluconate | 25 ppm copper citrate |
| 24 | not added | | 0 | 23 | 19 |
| 25 | Chlorhexidine gluconate | 2ppm | 32 | 93 | 89 |
| 26 | Chlorhexidine hydrochloride | 2ppm | 33 | 93 | 87 |
| 27 | Cetyl pyridinium chloride | 2ppm | 41 | 98 | 93 |
| 28 | Benzethonium chloride | 3ppm | 28 | 96 | 93 |
| 29 | Benzalkonium chloride | 3ppm | 25 | 97 | 92 |
| 30 | Decalinium chloride | 3ppm | 24 | 96 | 92 |
| 31 | Alkyldiaminoethylglycine hydrochloride | 5ppm | 18 | 94 | 91 |
| 32 | Isopropylmethylphenol | 20ppm | 22 | 96 | 91 |

As seen from Table 5, the combined use of copper gluconate and copper citrate and the specific bactericide shown in Table 5 exhibits synergistic bactericidal activity, providing for continuing mouth odor suppression.

Experiment 6

A test tube having an internal volume of 22-23 ml was charged with 1 ml of an aqueous solution or ethanol solution containing the copper compound and plant solvent extract whose type and amount are reported in Table 6, 5 ppm of sodium lauryl sulfate, and 5 ppm of sodium monofluorophosphate (only ethanol in the case of Control), and 1.5 ml of 0.1M phosphate buffer was added to pH 7.5. Immediately after 0.5 ml of a 10 vol% ethanol aqueous solution containing 1 $\mu$g/ml of methyl mercaptan was added, the tube was closed with a silicone rubber plug and vigorously shaken for one minute. The tube was allowed to stand for 6 minutes at 37°C before a gas chromatographic syringe was punctured into the plug to introduce 5 ml of air into the tube. The tube was again vigorously shaken for 30 seconds. Then 5 ml of gas was collected from the head space in the tube through the syringe and immediately introduced into a gas chromatograph, Model 163 (manufactured by Hitachi Ltd., Japan). The integrated count of methyl mercaptan was measured and percent deodorization was calculated therefrom according to the following formula:

$$\text{percent deodorization} = (C - S)/C \times 100\%$$

wherein C is an integrated count of Control (ethanol) and S is an integrated count of a sample.

Table 6

| Plant solvent extract 0.01wt% added | | Deodorization (%) | | |
|---|---|---|---|---|
| Sample No. | Type | No copper added | Copper gluconate 0.0025wt% | Copper citrate 0.0025wt% |
| Control | ethanol | - | 39 | 34 |
| Labiatae family plant | | | | |
| 33 | Sage (water extract) | 25 | 96 | 93 |
| 34 | Sage (acetone extract) | 26 | 97 | 94 |
| 35 | Rosemary (ethanol extract) | 30 | 100 | 96 |
| 36 | Rosemary (hexane extract) | 29 | 100 | 95 |
| 37 | Thyme (30% ethanol extract) | 31 | 100 | 97 |
| 38 | Scutellaria baicalensis Georgi (water extract) | 27 | 94 | 90 |
| 39 | Perilla (water extract) | 20 | 91 | 87 |
| 40 | Oregano (hexane extract) | 25 | 96 | 93 |
| Myrtaceae family plant | | | | |
| 41 | Clove (water extract) | 30 | 100 | 97 |
| 42 | Clove (acetone extract) | 30 | 100 | 96 |
| 43 | Eucalyptus (acetone extract) | 15 | 90 | 88 |
| 44 | Allspice (ethanol extract) | 20 | 93 | 89 |
| 45 | Myrtaceae Psidium gua (ethanol extract) | 25 | 97 | 95 |
| 46 | Rhodomyrtus (water extract) | 23 | 95 | 91 |

As seen from Table 6, a solution containing a solvent extract of a labiatae or myrtaceae family plant in combination with copper gluconate and copper citrate exerts a substantial deodorizing effect with respect to methyl mercaptan.

EXAMPLES

Examples of the present invention are given below by way of illustration and not by way of limitation. In the examples, all percents are percents by weight.

| Example 1: Toothpaste | |
| --- | --- |
| Aluminum hydroxide | 43% |
| Glycerin | 20% |
| Sodium carboxymethyl cellulose | 2% |
| Sodium lauryl sulfate | 2% |
| Flavor | 1% |
| Sodium saccharin | 0.1% |
| Sodium N-lauroyl sarcosinate | 0.2% |
| Sodium monofluorophosphate | 0.1% |
| Copper gluconate | 0.01% |
| Water | balance |
| Total | 100.0% |

| Example 2: Toothpaste | |
| --- | --- |
| Calcium secondary phosphate dihydrate | 45% |
| Titanium oxide | 0.3% |
| Sodium carboxymethyl cellulose | 0.3% |
| Sodium lauryl sulfate | 1.5% |
| Carrageenan | 0.8% |
| Sorbitol | 20% |
| Propylene glycol | 2% |
| Flavor | 1% |
| Sodium monofluorophosphate | 0.1% |
| Copper gluconate | 0.02% |
| Water | balance |
| Total | 100.0% |

| Example 3: Toothpaste | |
| --- | --- |
| Aluminum hydroxide | 42% |
| Glycerin | 10% |
| Sorbitol | 10% |
| Sucrose monolaurate | 2% |
| Sodium carboxymethyl cellulose | 1% |
| Carrageenan | 0.5% |
| Flavor | 0.8% |
| Sodium lauryl sulfate | 1% |
| Stannous fluoride | 0.05% |
| Copper gluconate | 0.01% |
| Water | balance |
| Total | 100.0% |

| Example 4: Toothpaste | |
|---|---|
| Calcium secondary phosphate dihydrate | 40% |
| Sorbitol | 10% |
| Sodium carboxymethyl cellulose | 1% |
| Sodium lauryl sulfate | 1% |
| Dextranase | 0.01% |
| Sodium saccharin | 0.1% |
| Sodium fluoride | 0.02% |
| Copper gluconate | 0.02% |
| Flavor | 1% |
| Water | balance |
| Total | 100.0% |

| Example 5: Toothpaste | |
|---|---|
| Calcium secondary phosphate dihydrate | 40% |
| Glycerin | 20% |
| Sodium carboxymethyl cellulose | 2% |
| Sodium lauryl sulfate | 1.2% |
| Sodium N-lauroyl sarcosinate | 0.2% |
| Sodium monofluorophosphate | 0.1% |
| Flavor | 1% |
| Sodium saccharin | 0.1% |
| Copper gluconate | 0.01% |
| Water | balance |
| Total | 100.0% |

| Example 6: Toothpaste | |
|---|---|
| Calcium secondary phosphate dihydrate | 40% |
| Sorbitol | 10% |
| Sodium carboxymethyl cellulose | 1% |
| Sodium cetyl sulfate | 1% |
| Dextranase | 0.01% |
| Sodium saccharin | 0.1% |
| Sodium fluoride | 0.02% |
| Copper gluconate | 0.02% |
| Flavor | 1% |
| Water | balance |
| Total | 100.0% |

EP 0 321 180 B1

| Example 7: Toothpaste | |
|---|---|
| Precipitated anhydrous amorphous silica | 30% |
| Sorbitol | 21% |
| Propylene glycol | 2.0% |
| Sodium carboxymethyl cellulose | 1.0% |
| Sodium saccharin | 0.1% |
| Flavor | 1.0% |
| Sodium lauryl sulfate | 1.5% |
| Copper gluconate | 0.005% |
| Sodium monofluorophosphate | 0.1% |
| Water | balance |
| Total | 100.0% |

| Example 8: Toothpaste | |
|---|---|
| Precipitated anhydrous amorphous silica | 30% |
| Sorbitol | 21% |
| Sodium carboxymethyl cellulose | 1.0% |
| Propylene glycol | 2.0% |
| Sodium saccharin | 0.1% |
| Flavor | 1.0% |
| Sodium lauryl sulfate | 1.5% |
| Copper gluconate | 0.0025% |
| Copper citrate | 0.0025% |
| Sodium monofluorophosphate | 0.1% |
| Water | balance |
| Total | 100.0% |

| Example 9: Toothpaste | |
|---|---|
| Precipitated zirconosilicate | 30% |
| Sorbitol | 21% |
| Propylene glycol | 2.0% |
| Sodium carboxymethyl cellulose | 1.0% |
| Sodium saccharin | 0.1% |
| Flavor | 1.0% |
| Sodium lauryl sulfate | 1.5% |
| Copper gluconate | 0.005% |
| Sodium monofluorophosphate | 0.1% |
| Water | balance |
| Total | 100.0% |

14

| Example 10: Toothpaste | |
|---|---|
| Zeolite | 20% |
| Calcium secondary phosphate | 20% |
| Sorbitol | 18% |
| Glycerin | 5.0% |
| Sodium carboxymethyl cellulose | 1.0% |
| Sodium saccharin | 0.1% |
| Flavor | 1.1% |
| Sodium lauryl sulfate | 1.5% |
| Copper gluconate | 0.005% |
| Sodium monofluorophosphate | 0.1% |
| Water | balance |
| Total | 100.0% |

Preferred fluorine compounds have some water solubility and may generally be ionic in character.

## Claims

1. An oral cavity composition comprising in admixture, copper gluconate, a soluble fluorine compound, and an alkali metal salt of an alkyl sulfate having 8 to 18 carbon atoms; wherein said copper gluconate, fluorine compound and alkali metal salt constitute a synergistic mixture for mouth odor suppression.

2. A composition of claim 1 which contains, based on the total weight of the composition, 0.00001 to 2% by weight of copper gluconate,
   0.001 to 1% by weight of the fluorine compound, and
   0.0001 to 3% by weight of the alkali metal salt of alkyl sulfate.

3. A composition of claim 1 or 2 which further comprises at least one member selected from precipitated amorphous silicas and silicates.

4. A composition of claim 3 wherein the precipitated amorphous silica and silicate have a specific surface area of up to 120 $m^2$/g as measured by the BET method using nitrogen gas.

5. A composition of any preceding claim which further comprises at least one flavour selected from cardamom oleo-resin, laurel oleo-resin, nutmeg oleo-resin, pimento oleo-resin, fennel oleo-resin, anise oleo-resin, bergamot oil, anethole, orange oil, lemon oil, and lime oil.

6. A composition of any preceding claim which further comprises at least one bactericide selected from chlorohexidine gluconate, chlorohexidine hydrochloride, cetyl pyridinium chloride, benzethonium chloride, benzalkonium chloride, decalinium chloride, alkyldiaminoethylglycine hydrochloride, and isopropylmethylphenol.

7. A composition of any preceding claim which further comprises a solvent extract from a labiatae or myrtaceae family plant.

8. A composition for cosmetic use comprising an oral cavity composition according to any preceding claim and including one or more substances suitable for oral use selected from adjuvants, fillers, carriers and abrasives.

9. A process for producing an oral cavity composition according to any preceding claim comprising a step of mixing together copper gluconate, a fluorine compound, and an alkali metal salt of an alkyl sulfate having 8 to 18 carbon atoms.

10. A process for giving an oral cavity composition mouth odour suppressant properties comprising incorporating copper gluconate, a soluble fluorine compound, and an alkali metal salt of an alkyl sulfate having 8 to 18 carbon atoms in the composition; wherein said copper gluconate, fluorine compound and alkali metal salt constitute a synergistic mixture for mouth odor supression.

**Revendications**

1. Composition buccale comprenant, en mélange, du gluconate de cuivre, un composé soluble de fluor, et un sel d'un métal alcalin d'un sulfate d'alkyle ayant 8 à 18 atomes de carbone, où lesdits gluconate de cuivre, composé de fluor et sel d'un métal alcalin constituent un mélange synergique pour la suppression des odeurs buccales.

2. Composition de la revendication 1 qui contient, en se basant sur le poids total de la composition,
0,00001 à 2% en poids de gluconate de cuivre,
0,001 à 1% en poids du composé de fluor et
0,0001 à 3% en poids du sel d'un métal alcalin de sulfate d'alkyle.

3. Composition de la revendication 1 ou 2 qui contient de plus au moins un élément choisi parmi des silices amorphes précipitées et silicates.

4. Composition de la revendication 3 où la silice amorphe précipitée et le silicate ont une aire superficielle spécifique pouvant atteindre 120 m$^2$/g en mesurant par la méthode BET en utilisant de l'azote gazeux.

5. Composition selon toute revendication précédente, qui contient de plus au moins un arôme choisi parmi une oléo-résine de cardamome, une oléo-résine de laurier, une oléo-résine de muscade, une oléo-résine de piment, une oléo-résine de fenouil, une oléo-résine d'anis, de l'essence de bergamote, de l'anéthol de l'essence d'orange, de l'essence de citron et de l'essence de lime.

6. Composition selon toute revendication précédente, qui contient de plus au moins un bactéricide choisi parmi le gluconate de chlorohexidine, le chlorhydrate de chlorohexidine, le chlorure de cétyl pyridinium, le chlorure de benzéthonium, le chlorure de benzalkonium, le chlorure de décalinium, le chlorhydrate d'alkyldiaminoéthylglycine et l'isopropylméthylphénol.

7. Composition selon toute revendication précédente qui contient de plus un solvant extrait d'une famille de plantes des labiacées ou des myrtacées.

8. Composition pour usage cosmétique comprenant une composition buccale selon toute revendication précédente, contenant une ou plusieurs substances appropriées à un usage oral,choisies parmi des adjuvants, charges, véhicules et abrasifs.

9. Procédé de production d'une composition buccale selon toute revendication précédente,comprenant une étape de mélanger ensemble du gluconate de cuivre, un composé de fluor et un sel d'un métal alcalin d'un sulfate d'alkyle ayant 8 à 18 atomes de carbone.

10. Procédé pour donner à une composition buccale des propriétés de suppression de l'odeur de la bouche, comprenant l'incorporation de gluconate de cuivre, d'un composé soluble de fluor et d'un sel d'un métal alcalin d'un sulfate d'alkyle ayant 8 à 18 atomes de carbone dans la composition ; où lesdits gluconate de cuivre, composé de fluor et sel d'un métal alcalin constituent un mélange synergique pour la suppression de l'odeur buccale.

**Patentansprüche**

1. Zusammensetzung für die Mundhöhle, in Mischung enthaltend Kupferglukonat, eine lösliche Fluorverbindung und ein Alkalimetallsalz eines Alkylsulfats mit 8 bis 18 C-Atomen; worin die genannten Kupferglukonat, Fluorverbindung und Alkalimetallsalz eine synergistische Mischung zur Unterdrückung von Mundgeruch darstellen.

2. Zusammensetzung nach Anspruch 1, die, auf der Basis des Gesamtgewichts der Zusammensetzung,
0,00001 bis 2 Gew.-% Kupferglukonat,
0,001 bis 1 Gew.-% der Fluorverbindung und
0,0001 bis 3 Gew.-% des Alkalimetallsalzes von Alkylsulfat enthält.

16

**3.** Zusammensetzung nach Anspruch 1 oder 2, die weiterhin zumindest einen aus ausgefällten amorphen Kieselsäuren und Silikaten ausgewählten Bestandteil umfaßt.

**4.** Zusammensetzung nach Anspruch 3, worin die ausgefällten amorphen Kieselsäuren und Silikate eine spezifische Oberfläche von bis zu 120 m$^2$/g, gemessen gemäß dem BET-Verfahren unter Verwendung von Stickstoffgas, aufweisen.

**5.** Zusammensetzung nach einem der vorhergehenden Ansprüche, die weiterhin zumindest einen Geschmacksstoff umfaßt, der aus Kardamom-Ölharz, Lorbeer-Ölharz, Muskatnuß-Ölharz, Piment-Ölharz, Fenchel-Ölharz, Anis-Ölharz, Bergamottöl, Anethol, Orangenöl, Zitronenöl und Limonenöl ausgewählt ist.

**6.** Zusammensetzung nach einem der vorhergehenden Ansprüche, die weiterhin zumindest ein Bakterizid umfaßt, das aus Chlorhexidinglukonat, Chlorhexidinhydrochlorid, Cetylpyridiniumchlorid, Benzethoniumchlorid, Benzalkoniumchlorid, Decaliniumchlorid, Alkyldiaminoäthylglycinhydrochlorid und Isopropylmethylphenol ausgewählt ist.

**7.** Zusammensetzung nach einem der vorhergehenden Ansprüche, die weiterhin einen Lösungsmittelextrakt von einer Pflanze der Familie labiatae oder myrtaceae umfaßt.

**8.** Zusammensetzung zur kosmetischen Verwendung, die eine Zusammensetzung für die Mundhöhle nach einem der vorhergehenden Ansprüche umfaßt und eine oder mehrere für die orale Verwendung geeignete Substanz(en) enthält, die aus Hilfs-, Füll-, Träger- und Schleifmitteln ausgewählt ist/sind.

**9.** Verfahren zur Herstellung einer Zusammensetzung für die Mundhöhle nach einem der vorhergehenden Ansprüche, das einen Schritt des Zusammenmischens von Kupferglukonat, einer Fluorverbindung und eines Alkalimetallsalzes eines Alkylsulfats mit 8 bis 18 C-Atomen umfaßt.

**10.** Verfahren, um einer Zusammensetzung für die Mundhöhle Eigenschaften der Unterdrückung von Mundgeruch zu verleihen, welches das Einbringen von Kupferglukonat, einer löslichen Fluorverbindung und eines Alkalimetallsalzes eines Alkylsulfates mit 8 bis 18 C-Atomen in die Zusammensetzung umfaßt; worin die genannten Kupferglukonat, Fluorverbindung und Alkalimetallsalz eine synergistische Mischung zum Unterdrücken von Mundgeruch darstellen.